# EUROPÄISCHE PATENTSCHRIFT

(11) **EP 2 366 411 B1**
(45) Veröffentlichungstag und Bekanntmachung des Hinweises auf die Patenterteilung: **06.05.2015**
(21) Anmeldenummer: 11158812.5
(22) Anmeldetag: 18.03.2011
(51) Int. Cl.: A61L 2/07, A61L 2/26, A61L 2/28

(54) **Sterilisator**
Steriliser
Stérilisateur

(30) Priorität: 18.03.2010 DE 102010016017
(43) Veröffentlichungstag der Anmeldung: 21.09.2011
(73) Patentinhaber: MMM Münchener Medizin Mechanik GmbH, 82152 Planegg (DE)
(72) Erfinder: Eibl, Robert, 82539, Schönsee (DE); Nickel, Katrin, 82377, Penzberg (DE); Krotz, Martin, 81371, München (DE)
(74) Vertreter: Wittmann, Ernst-Ulrich

(56) Entgegenhaltungen:
- EP-A1- 0 982 039
- EP-A1- 1 468 701
- WO-A1-00/06211
- US-B1- 6 323 032

## Beschreibung

Die Erfindung betrifft einen Sterilisator mit einer Sterilisierkammer und mit einer Prüfeinrichtung zur Prüfung der Effektivität eines Sterilisationsprozesses, die einen Prüfkörper und eine Sonde umfasst, wobei die Sonde im Betrieb mit dem Prüfkörper verbunden ist. Des Weiteren betrifft die Erfindung ein Verfahren zum Betreiben des Sterilisators und eine Vorrichtung zur Prüfung der Effektivität eines Sterilisationsprozesses.

Sterilisatoren der eingangs genannten Art sind aus dem Stand der Technik bekannt und werden vornehmlich aber nicht ausschließlich im Gesundheitswesen und angrenzenden Bereichen verwendet, um die weitgehende Keimfreiheit eines zu sterilisierenden Gegenstands - auch Sterilisiergut genannt - zu gewährleisten. Bei diesen Gegenständen handelt es sich in der Regel um medizinische Geräte oder Instrumente, Kleidung und dergleichen.

Zur Sicherstellung der Keimfreiheit und zur Qualitätskontrolle muss die Effektivität bzw. Wirksamkeit des Sterilisationsprozesses überprüft werden. Diese Überprüfung kann nicht am Sterilisiergut erfolgen, da dieses dann für die vorgesehene Anwendung unbrauchbar gemacht wird, weil durch jede direkte Überprüfung auf Sterilität das Sterilisiergut unsteril wird. Daher erfolgt die Prüfung der Effektivität eines Sterilisationsprozesses üblicherweise mittels einer Prüfeinrichtung mit einem Prüfkörper indirekt während des Sterilisationsprozesses durch die Messung physikalischer Parameter und/oder mittels eines chemischen Indikators, beispielsweise durch Farbumschlag des Indikators. Damit kann die Messung wichtiger Parameter für die Wirksamkeit nicht direkt am Sterilisiergut sondern an einem Prüfkörper erfolgen, dessen Eigenschaften dem des Sterilisierguts möglichst nahe kommt, beispielsweise hinsichtlich Luftentfernung oder Transport des die Sterilisation bewirkenden Agens an dessen Oberflächen oder der Temperatur des Sterilisierguts.

Zur Sterilisation sind beispielsweise Dampfsterilisationsverfahren bekannt, wobei diese Verfahren in einer Sterilisierkammer durchgeführt werden. Bei diesen Sterilisationsverfahren unterscheidet man im Wesentlichen drei Phasen. In der ersten Phase, der sogenannten Entlüftungsphase, wird der Sterilisierkammer Luft entzogen und durch Dampf ersetzt. In Phase zwei erfolgt die Sterilisation mittels Dampf, wobei der Dampf unter einem bestimmten Druck mit einer bestimmten Temperatur und für eine bestimmte Zeit in der Sterilisierkammer gehalten wird. In der dritten Phase erfolgt eine Trocknung, während der das Kondensat durch Absaugung aus der Sterilisierkammer entfernt wird.

Man kennt hierbei verschiedene Verfahren zur Überprüfung der Effektivität der Dampfdurchdringung, beispielsweise den chemischen oder den elektronischen Bowie&Dick-Test, die in der Regel einmal täglich als Leertest oder Routineüberprüfung durchgeführt werden.

Beim chemischen Bowie&Dick-Test wird ein chemischer Indikator in ein entweder als poröses System oder in ein als Hohlkörper-System ausgestalteten Prüfkörper eingelegt, der sich in der Sterilisierkammer befindet und so dem Sterilisationsprozess ausgesetzt wird. Die ausreichende Luftentfernung und Dampfdurchdringung während des Sterilisationsprozesses wird dabei durch einen Farbumschlag am Indikator ermittelt. Die Anforderungen an ein Bowie&Dick-Testsystem sind in entsprechenden Normen definiert. Solche Systeme werden üblicherweise als Einmal-Systeme ausgeführt.

Beim elektronischen Bowie&Dick-Test wird ein Prüfkörper bestehend aus einem Hohlkörper-System, das sich in der Sterilisierkammer befindet, mit thermischen Massen dem Sterilisationsprozess ausgesetzt. Durch eine oder mehrere Temperaturmessungen am Testsystem kann entschieden werden, ob der Sterilisationsprozess erfolgreich war. Die Datenübertragung kann wahlweise direkt über Funk nach außen erfolgen oder sie werden nach dem Sterilisationsprozess aus einem im System vorgesehenen Datenspeicher, beispielsweise einem Datenlogger, abgerufen. Auch dieses System hat den genormten Anforderungen an den Bowie&Dick-Test zu genügen. Ein solches System ist aus der DE 20 2006 006 926 U1 bekannt.

Die US 6,323,032 B1 zeigt einen solchen Prüfköper wie er bei den verschiedenen Bowie&Dick-Tests eingesetzt werden kann. Der Prüfköper ist hierbei während der Tests in der Sterilisierkammer angeordnet.

Des Weiteren wird häufig bei jeder Sterilisationscharge ein Sterilisationstest in Form eines zusätzlichen Routinetests durchgeführt. Hierbei werden verschiedene Tests eingesetzt, die nachfolgend beschrieben werden. Eine Art von Test erfolgt durch ein Chargenkontrollsystem, bei dem ein chemischer Indikator in einem Prüfkörper eingebracht wird, der entweder als poröses System oder in ein Hohlkörper-System ausgestaltet ist, und der in der Sterilisatorkammer dem Sterilisationsprozess ausgesetzt wird. Die ausreichende Luftentfernung und Dampfdurchdringung des Sterilisationsprozesses wird durch einen Farbumschlag am Indikator ermittelt. Die Anforderungen an Chargenkontrollsysteme sind zur Zeit nicht genormt, üblicherweise werden jedoch die Geometrien der Testsysteme mit den Geometrien der realen Beladung abgeglichen.

Bei einer anderen Art der Überprüfung wird bei jeder Sterilisationscharge ein Chargenkontrollsystem eingesetzt, bei dem ein als Hohlkörper-System ausgestalteter Prüfköper mit thermischen Massen dem Sterilisationsprozess ausgesetzt wird. Durch eine oder mehrere Temperaturmessungen am Testsystem kann entschieden werden, ob der Sterilisationsprozess erfolgreich war. Die Datenübertragung kann wahlweise direkt über Funk nach außen erfolgen oder sie werden nach dem Prozess aus einem Datenlogger abgerufen. Solche Systeme werden üblicherweise gegen übliche genormte Testladungen qualifiziert.

Eine Überprüfung der Effektivität bei jeder Sterilisationscharge kann auch durch ein Luftnachweisgerät, einem sogenannten Airdetector, nach DIN EN 285 erfolgen, in der allgemein und ohne Vorgabe der Technologie die Anforderungen bezüglich der Erkennung der Auswirkung von Restluft, Luftleckagen oder nichtkondensierbaren Gasen im zugeführten Dampf an einem Wäschepaket beschrieben werden. Bei gängigen Systemen wird ein separater Behälter mit Sterilisierkammerdampf durchströmt. Dabei wird der unterschiedliche Temperaturanstieg bei Dampf bzw. Dampf-/Luftgemisch ausgewertet. Generell sind solche Systeme nicht für Hohlkörper geeignet.

Eine weitere Art der Effektivität der Wirksamkeit des Sterilisationsprozesses bei jeder Sterilisationscharge ist ein sog. IPC - In-Process-Control - , bei dem der Dampf aus der Sterillsatlonskammer während der Sterilisierphase in einem Teilstrom entnommen und in einen rohrförmigen Prüfkörper, beispielsweise einem Kapillarrohr, geleitet wird. Durch eine Kühleinrichtung vor dem Prüfkörper kondensiert der Dampf, und das Dampf-/Luft-Gemisch wird dem Prüfkörper zugeführt, in dem dann die Zusammensetzung des Dampfes, insbesondere die im Dampf enthaltenen nichtkondensierbaren Gase, durch Lichtschranken ermittelt werden. Ein solches System ist beispielsweise in der DE 36 36 716 C2 gezeigt. Das gemessene Kondensat-Gasanteil-Verhältnis ist dabei ein Maß für die Qualität des Sterilisationsprozesses. Das System ist jedoch aufwendig und empfindlich und aufgrund seiner Konstruktion nicht für Hohlkörper geeignet.

Im Stand der Technik sind des weiteren ein Dampfsterilisator aus der EP 0 982 039 A1 bekannt, der insbesondere dazu dient Hohlkörper zu sterilisieren. Ferner ist ein Erfassungs- und Steuerungssystem in Echtzeit für ein antimikrobielles Verfahren aus der WO 00/06211 und ein Prüfkörper, insbesondere zur Überprüfung des Penetrationseigenschaften eines Sterilisiermittel in Sterilisationsprozessen aus der EP 1 468 701 A1 bekannt. Darüber hinaus ist im Stand auch eine System zum Prüfen von Sterilisatoren aus der US 6,323,032 B1 bekannt.

Der vorliegenden Erfindung liegt die Aufgabe zugrunde, einen Sterilisator und ein Verfahren der eingangs genannten Art anzugeben, bei der eine verbesserte Handhabung und Einsatzfähigkeit gegeben ist.

Erfindungsgemäß wird die voranstehende Aufgabe durch einen Sterilisator mit den Merkmalen des Patentanspruchs 1 gelöst. Danach ist der in Rede stehende Sterilisator derart ausgestaltet und weitergebildet, dass der Prüfkörper außerhalb und die Sonde mindestens teilweise innerhalb der Sterilisierkammer angeordnet ist und dass die Prüfeinrichtung mit der Sterilisierkammer fest verbindbar ist. Des Weiteren wird die Aufgabe durch ein entsprechendes Verfahren gemäß Patentanspruch 9 gelöst.

Erfindungsgemäß ist erkannt worden, dass man in Abkehr zu dem im Stand der Technik bekannten Prüfeinrichtungen einen schnelleren und sicheren Einsatz der Prüfeinrichtung dadurch erreicht, dass die Prüfeinrichtung derart mir dem Sterilisator verbunden ist, dass der Prüfkörper außerhalb und zumindest ein Teil der Sonde innerhalb der Sterilisierkammer angeordnet sind, wobei die Sonde mit dem Prüfkörper verbunden ist. Beispielsweise kann die Sonde durch einen an der Sterilisierkammer befindlichen Stutzen geführt werden und der Prüfkörper, vorzugsweise mit einem an diesen angebrachten Temperatursensor, am Ende des Stutzens außerhalb der Sterilisierkammer versch raubt werden. Die Prüfeinrichtung könnte mit der Sterilisierkammer fest verbunden sein. Diese feste Verbindung könnte jedoch insofern lösbar ausgestaltet sein, um einen Austausch von Verschleißteilen gewährleisten zu können. Anders als bei bekannten Systemen, die am Sterilisator angebracht sind, wird weder ein Volumenstrom des Dampf-Gas-Gemisches abgezweigt, noch ist eine störanfällige Messung nichtkondensierbarer Gase über eine Lichtschranke notwendig. Stattdessen kann eine thermische Messung am Prüfkörper erfolgen. Die Sonde, welche im Betrieb mit dem Prüfkörperverbunden ist, kann als Hohlkörper zur Leitung von Dampf und/oder Agens und/oder Gasen ausgestaltet sein. Dieser Hohlkörper kann eine aus Kunststoff bestehende Leitung sein und/oder an dem in der Sterilisierkammer angeordneten Ende offen sein, um so Dampf, Agens, und/oder Temperatur zu dem Prüfkörper zu leiten. Insbesondere kann die Sonde so ausgestaltet werden, beispielsweise durch mehrfaches Einrollen, dass der Sterilisator zur Prüfung der Effektivität von Sterilisationsprozesse bei Medizinprodukten und anderen Sterilisiergütern geeignet ist, welche aufgrund ihrer Geometrien oder sonstigen Eigenschaften besondere Anforderungen an den Sterilisationsprozess stellen. Ein solcher "qualifizierter" Nachweis der Effektivität von Sterilisationsprozessen, insbesondere bei der Sterilisation von Hohlkörpern, ist bei den bekannten Systemen, welche am Sterilisator angebracht sind, derzeit nicht möglich.

Während der ersten Phase eines Sterilisationsprozesses könnte somit durch die Fraktionierungen die in der Sonde befindliche Restluft durch das Agens (z.B. Dampf) verdünnt und auf das Ende der Sonde zurückgedrängt. Dort wäre die Sonde mit dem Prüfkörper verbunden und ein Temperaturfühler würde in das Sonden-Ende hereinragen. Dadurch, dass das Agens auch den anfangs noch kalten Prüfkörper aufheizen würde, könnte weiterhin Restluft aus der Kammer, Leckagen und nicht-kondensierbare Gase an das Sonden-Ende gelangen und könnten dort durch den Temperaturfühler aufgrund von Temperaturabweichungen gegenüber einem "idealen" Prozess detektiert werden. Bei Systemen, die in die Sterilisatorkammer eingelegt werden (z.B. Chemo-Indikatoren), ist dieser Effekt jedoch dadurch beschränkt, dass das Agens den Prüfkörper nicht nur über den Weg der Sonde aufheizen kann, sondern auch direkt von außen. Erfindungsgemäß entfällt diese Beschränkung dadurch, dass der Prüfkörper selbst außerhalb der Kammer angeordnet ist.

Darüber hinaus müssen die Bowie&Dick-Tests einmal täglich durchgeführt werden und es bestehen DIN EN Normen für die Qualifizierung der Bowie&Dick-Testsysteme. Bestehende Systeme, die am Sterilisator angebracht sind, genügen diesen Vorgaben nicht. Sie können also nur als zusätzliche Chargenkontrollsysteme verwendet werden.

Der Unterschied zum Stand der Technik besteht im Hinblick auf chemische und elektronische Bowie&Dick-Tests und anderen Vorrichtungen, welche der Anwender in die Sterilisierkammer einbringen muss, darin, dass die Prüfeinrichtung sich am Sterilisator befindet.

Neben der verbesserten Handhabung und Einsatzfähigkeit könnte der Erfindung also die zusätzliche Aufgabe zugrunde legen, einen Sterilisator zu schaffen, mit welchem die vorgeschriebenen täglichen Bowie&Dick-Test erfüllt werden können und mit dem zusätzliche Chargenkontrollen durchgeführt werden können, ohne dass neben der einmaligen Investition eines Sterilisators weitere Aufwendungen für die Testsysteme getätigt werden müssen.

Zudem werden wesentliche Nachteile bei der Handhabung der beschriebenen Chargenkontrollsysteme mit chemischen Indikatoren oder anderen Vorrichtungen vermindert, die der Anwender vor Beginn des Sterilisationsprozesses in die Sterilisierkammer einlegen muss. Dies ist insbesondere der Fall, weil es sich hierbei üblicherweise um Einmal-Systeme handelt und es daher zu Verwechslungen der Prüfkörper und Indikatoren kommen kann, insbesondere wenn der Anwender mehrere Sterilisatoren betreibt, und weil für unterschiedliche Beladungen unterschiedliche Chargenkontrollsysteme eingesetzt werden müssen oder weil der Anwender schlichtweg vergessen kann, die chemischen Indikatoren oder anderen Vorrichtungen in die Sterilisierkammer zu legen. Außerdem muss der Anwender zumindest bei Beginn und Ende des Prüfverfahrens anwesend sein, um die jeweilige Prüfung durchführen zu können.

Außerdem kann die Prüfeinrichtung relativ schnell wieder "abkühlen", um für weitere Tests zur Verfügung zu stehen, was allein durch die Anordnung des Prüfkörpers außerhalb der Sterilisierkammer gewährleistet wird.

In vorteilhafter Weise könnte eine Kühleinrichtung zur Kühlung des Prüfkörpers vorgesehen sein. Wenn der Prüfkörper gekühlt wird, dann könnte ein Zurückführen der Temperatur auf eine Ausgangstemperatur schneller erfolgen, so dass eine schnellere Wiedereinsetzbarkeit und somit eine verbesserte Handhabbarkeit erreicht wird. Durch das Abkühlen auf eine Ausgangstemperatur werden reproduzierbare und sichere Ergebnisse erreicht, so dass man für einen kontinuierlichen Betrieb nur noch ein System benötigt wird, was bei den im Stand der Technik bekannten Systemen nicht möglich ist. Eine solche Prüfeinrichtung mit Kühlung könnte auch für sich gesehen erfindungsgemäß sein.

Die Kühlung wird vorzugsweise nach Beendigung des vorherigen und vor Beginn des nächsten Sterilisationsprozesses durchgeführt. Die Kühlung könnte jedoch bereits früher beginnen, beispielsweise bereits während der Endphase des vorherigen und/oder während des Beginns des nachfolgenden Sterilisationsprozesses. Hierdurch könnte eine noch schnellere Wiedereinsetzbarkeit erreicht werden.

Im Rahmen einer kostengünstigen Ausgestaltung könnte die Kühleinrichtung als passive Kühleinrichtung ausgestaltet sein, welche vorzugsweise durch freie Konvektion erfolgen könnte. Dies könnte durch das Vorsehen von geeigneten Maßnahmen am oder im Prüfkörper verbessert werden.

Hinsichtlich einer besonders effektiven Ableitung der Wärme am Prüfkörper könnte die Kühleinrichtung als aktive Kühleinrichtung zur geregelten Kühlung des Prüfkörpers ausgestaltet sein und eine Steuerungseinrichtung vorgesehen sein, die mit der aktiven Kühleinrichtung zur Steuerung der Kühlleistung verbindbar sein könnte. Unter Regelung wird in diesem Zusammenhang auch eine Steuerung erfasst.

Im Hinblick auf eine einfache und gegebenenfalls kostengünstige Kühlung könnte die aktive Kühleinrichtung ein Kühlmedium, beispielsweise Wasser- und/oder Luft, und/oder eine Kühlung durch Peltier-Elemente, also eine thermo-elektrische Kühlung, und/oder eine Einrichtung für die Erzeugung einer erzwungenen Konvektion umfassen. Da der Prüfkörper vorzugsweise außerhalb der Sterilisierkammer angeordnet ist, würde man in vorteilhafter Weise keine besonderen Isoliermaßnahmen der aktiven Kühleinrichtung benötigen.

In vorteilhafter Weise könnte mittels der Steuerungseinrichtung die Temperatur des Prüfkörpers einstellbar sein. Hierdurch wäre gewährleistet, dass eine definierte Temperatur einstellbar ist und somit sichergestellt ist, dass genau diese Temperatur des Prüfkörpers, vorzugsweise in einer definierten Zeit, erreicht wird. In weiter vorteilhafter Weise könnte das Prüfsystem und/oder ein damit verwendeter Sterilisator blockiert werden, wenn die definierte Temperatur des Prüfkörpers nicht erreicht ist.

Hinsichtlich einer wiederum einfachen Ausgestaltung des Sterilisators könnte die Sonde als Hohlkörper zur Leitung von Dampf und/oder Agens und/oder Gasen ausgestaltet sein. Dieser Hohlkörper könnte eine aus Kunststoff bestehende Leitung sein und/oder an dem in der Sterilisierkammer angeordneten Ende offen sein, um so Dampf, Agens, und/oder Temperatur zu dem Prüfkörper zu leiten.

Bei den bekannten physikalischen Systemen, bei denen eine entsprechende Vorrichtung in die Sterilisierkammer eingelegt wird, erfolgt die Detektierung von Restluft, Leckagen oder nichtkondensierbaren Gasen mittels einer Temperaturmessung: Am Ende eines als Hohlkörpersystem ausgestalteten Prüfkörpers befindet sich dazu ein Temperatursensor. Wird das Hohlkörpersystem ausreichend entlüftet und ausreichend von Dampf durchdrungen, so soll dies durch einen entsprechenden Temperaturverlauf angezeigt werden. Vorausgesetzt wird hier allerdings, dass der Wärmeeintrag ausschließlich durch den Dampf über den Weg des Hohlkörpersystems erfolgt. Da Wärme jedoch ebenso durch den Dampf von außen - unter Umgehung des Hohlkörpersystems - an den Prüfkörper und den Temperatursensor herangeführt wird, kann im Zweifelsfall nicht sicher entschieden werden, ob ein Temperaturanstieg durch Wärmeleitung von außen oder durch Dampfdurchdringung hervorgerufen wurde. Dies hängt entscheidend von der Konstruktion der Prüfseinrichtung ab. Somit muss der Prüfkörper mit dem Temperatursensor entweder groß genug gewählt oder entsprechend thermisch isoliert werden, um die Wärmeleitung von außen zu minimieren. Mit solchen Prüfsystemen sind heutige, filigrane Medizinprodukte in ihrem thermischen Verhalten daher nicht ausreichend nachzubilden.

Die vorliegende Erfindung umgeht dies, indem der Prüfkörper mit dem Temperatursensor nicht innerhalb der Kammer, sondern außerhalb der Kammer angeordnet ist. Dadurch erfolgt ein Temperaturanstieg während eines Zyklus durch Dampfdurchdringung über den Weg der Sonde. Der Temperatursensor kann also in vorteilhafter Weise die Temperatur des über die Sonde zum Prüfkörper transportierten Dampfs und/oder Agens und somit die Sterilisationstemperatur besonders genau messen, da er nicht zusätzlich direkt von dem in der Sterilisierkammer befindlichen Dampf beeinflusst wird.

Im Hinblick auf eine besonders sichere Ausgestaltung könnte die Sonde im oberen, unteren oder seitlichen Bereich der Sterilisierkammer angeordnet sein und somit in dem Bereich, in dem sich keine Aufnahme von zu sterilisierenden Gegenständen befindet. Somit kann der Sterilisator ohne weitere Vorsichtsmaßnahmen beladen werden, ohne dass die Sonde beschädigt wird.

Weiter erfindungsgemäß ist ein Verfahren zum Betreiben eines obig beschriebenen Sterilisators vorgesehen, das derart weitergebildet ist, dass die Prüfeinrichtung mittels einer Sterilisatorsteuerung automatisch mit der Wahl eines Sterilisierprogramms aktiviert wird, so dass ein Prüfprogramm gestartet wird. Erfindungsgemäß könnte somit bei jedem Ablauf eines Sterilisationsprozesses eine Prüfung "automatisch" durchgeführt werden. Beispielsweise können eine Routineprüfungen bzw. Leerprüfung bei der Auswahl und Start des jeweiligen Sterilisierprogramms automatisch aktiviert werden, ohne dass dies der Anwender berücksichtigen muss.

In vorteilhafter Weise könnten durch die Prüfeinrichtung während eines Prüfprogramms - sei es während einer Leerprüfung oder einer Chargenprüfung- ermittelten Daten an ein Chargendokumentationssystem übermittelt werden und/oder durch dieses ausgewertet werden. Die Ergebnisse der Prüfungen stünden dann nach Ablauf eines Sterilisationsprozesses zur Verfügung und können vorzugsweise zusammen mit den anderen "Chargendaten" ausgewertet und/oder gespeichert und/oder zur Qualitätssicherung archiviert werden.

In einer weiter vorteilhaften Ausgestaltung könnte durch die Aktivierung der Prüfeinrichtung mittels der Sterilisatorsteuerung ein vorgegebenes Programm, vorzugsweise eine Leerprüfung bzw. Routineprüfung des Sterilisators, zeitverzögert durchgeführt werden. Es könnte jedoch auch ein unmittelbarer Start des Programms nach der Aktivierung erfolgen. Alternativ oder zusätzlich könnte dies auch bei Aktivierung des Sterilisators erfolgen.

Des Weiteren könnte mittels der Sterilisiersteuerung eine Folge von Programmen einstellbar sein, die nach der Aktivierung automatisiert abläuft und/oder wobei eine Leerprüfung Teil der Folge ist. Somit könnte ein Anwender beispielsweise am Vortag einen kompletten Programmablauf mit Vakuumtest, Aufwärmprogramm und dem täglichen "Standard-Bowle-Dick-Test" bei leerer Sterilisierkammer wählen. Dieser Programmablauf- welcher bis zu mehreren Stunden dauern kann - würde dann zeitverzögert am Morgen des darauffolgenden Tags ablaufen, und die Ergebnisse der Routineprüfungen lägen dem Anwender bei "Arbeitsantritt" vor.

In weiter erfindungsgemäßen Weise ist eine Vorrichtung zur Prüfung der Effektivität eines Sterilisationsprozesses vorgesehen mit einer Prüfeinrichtung, wobei die Prüfeinrichtung einen Prüfkörper und eine Sonde umfasst, wobei die Sonde im Betrieb mit dem Prüfkörper verbunden ist, wobei eine Kühleinrichtung zur Kühlung des Prüfkörpers vorgesehen ist. Die Prüfeinrichtung könnte wie bereits beschrieben ausgestaltet sein.

Es gibt nun verschiedenen Möglichkeiten, die Lehre der vorliegenden Erfindung in vorteilhafter Weise auszugestalten und weiterzubilden. Dazu ist einerseits auf die den Patentansprüchen 1 und 9 nachgeordneten Patentansprüche und anderseits auf die nachfolgende Erläuterung eines bevorzugten Ausführungsbeispiels eines erfindungsgemäßen Sterilisators mit einer Prüfeinrichtung zur Prüfung der Effektivität eines Sterilisationsprozesses und eines entsprechenden erfindungsgemäßen Verfahrens zum Betreiben des Sterilisators anhand der Zeichnung zu verweisen. In Verbindung mit dieser Erläuterung des bevorzugten Ausführungsbeispiels anhand der Zeichnung werden auch im Allgemeinen bevorzugte Ausgestaltungen und Weiterbildungen der Lehre erläutert. In der Zeichnung zeigen
- Fig. 1: in einer schematischen Darstellung, ein Ausführungsbeispiel eines erfindungsgemäßen Sterilisators zur Prüfung der Effektivität eines Sterilisationsprozesses, in diesem Fall eines Dampfdurchdringungsprozesses und
- Fig. 2: in einer schematischen Darstellung, ein weiteres Ausführungsbeispiel eines erfindungsgemäßen Sterilisators zur Prüfung der Effektivität eines Sterilisationsprozesses, in diesem Fall eines Dampfdurchdringungsprozesses.

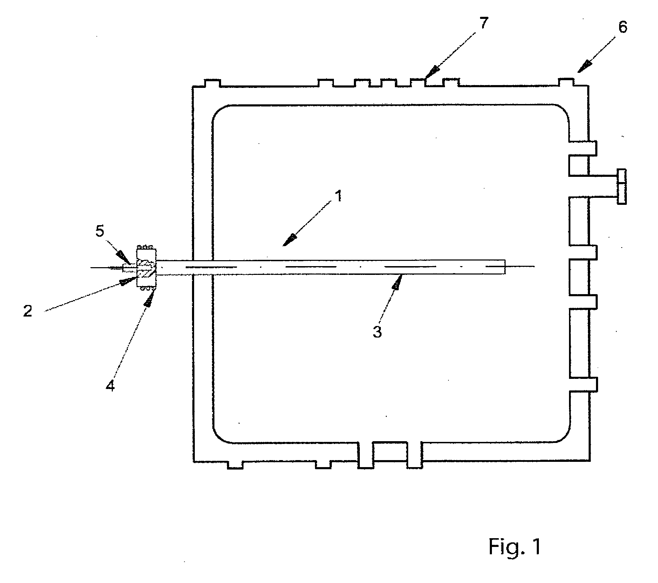

Fig. 1 zeigt einen Sterilisator, der eine Prüfeinrichtung 1 zur Prüfung der Effektivität eines Dampfdurchdringungsprozesses aufweist. Die Prüfeinrichtung 1 umfasst hierbei einen Prüfkörper 2 und eine Sonde 3. Die Sonde 3 ist im Betrieb mit dem Prüfkörper 2 verbunden.

Erfindungsgemäß ist der Prüfköper 2 außerhalb und die Sonde 3 mindestens teilweise innerhalb der Sterilisierkammer 7 angeordnet und ist die Prüfeinrichtung 1 mit der Sterilisierkammer 7 fest verbindbar. Des Weiteren ist eine - nicht dargestellte - Steuerungseinrichtung vorgesehen, die mit einer aktiven Kühleinrichtung 4 zur Steuerung der Kühlleistung verbindbar ist. Die Steuereinrichtung ist hierbei drahtlos mit der aktiven Kühleinrichtung 4 verbunden und ist körperlich nicht unmittelbar mit der aktiven Kühleinrichtung verbunden. In diesem Ausführungsbeispiel erfolgt die Kühlung des Prüfköpers 2 auf Raumtemperatur mittels eines Kühlmediums, nämlich mittels Wasser.

Die Sonde 3 ist in diesem Ausführungsbeispiel als weitgehend starrer Hohlkörper ausgestaltet, durch den Dampf zum Prüfkörper 2 geleitet wird. Ferner ist am Prüfkörper 2 ein Temperatursensor 5 angeordnet, der die vom Dampf erzeugte Temperatur misst.

Der Sterilisator 6 wird mittels eines Verfahrens betrieben, das vorsieht, dass die Prüfeinrichtung 1 mittels einer - ebenfalls nicht gezeigten - Sterilisiersteuerung automatisch mit der Wahl eines Sterilisierprogramms aktiviert wird, so dass ein Prüfprogramm gestartet wird. Durch die Prüfeinrichtung 1 werden während des Prüfprogramms Daten über das Prüfprogramm ermittelt und an ein - nicht gezeigtes - Chargendokumentationssystem übermittelt. Das Chargendokumentationssystem wertet diese Daten aus und archiviert sie zur Qualitätskontrolle.

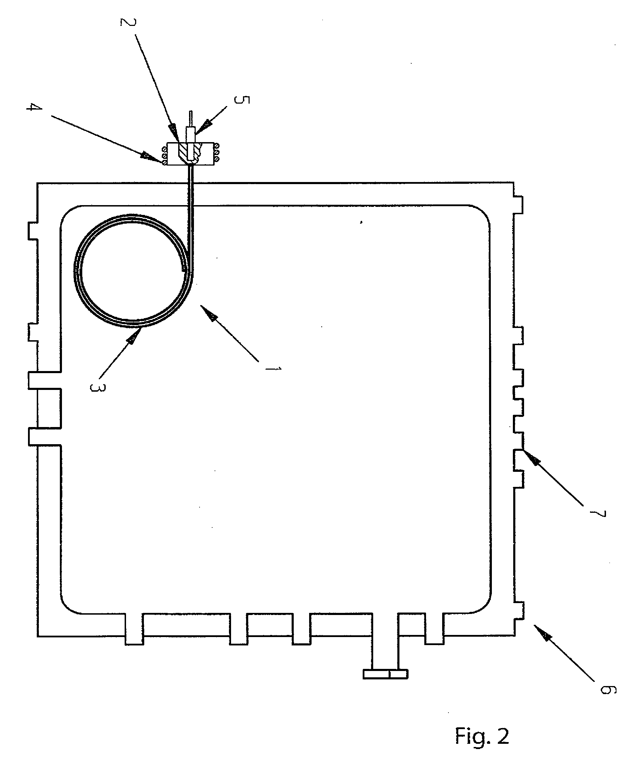

Fig. 2 zeigt ein weiteres Ausführungsbeispiel eines erfindungsgemäßen Sterilisators, der ähnlich mit dem in Fig. 1 gezeigten Sterilisator aufgebaut ist. Identische Teile sind hierbei mit identischen Bezugsziffern bezeichnet. Zur Vermeidung von Wiederholungen wird auf die Beschreibung des in Fig. 1 gezeigten Ausführungsbeispiel verwiesen.

Im Gegensatz zu dem in Fig. 1 gezeigten Ausführungsbeispiel ist die Sonde 3' nicht starr in die Kammer hereinragt sondern in einer zusammengerollten Form im unteren Bereich der Sterilisierkammer 7 angeordnet ist. Hierdurch wird zum Einen die Beladung der Sterilisierkammer 7 vereinfacht, zum Anderen kann derart der Nachweis für eine erfolgreiche Sterilisation von komplexeren Sterilisiergut verbessert werden, da der Durchtritt des Dampfes durch die Sonde 3' erschwert wird. Andere Formen der Sonde 3' sind ebenfalls denkbar,

Hinsichtlich weiterer Details wird zur Vermeidung von Wiederholungen auf die allgemeine Beschreibung verwiesen.

Schließlich sei daraufhingewiesen, dass das voranstehende Ausführungsbeispiel lediglich zur Erläuterung der beanspruchten Lehre dient, diese jedoch nicht auf das Ausführungsbeispiel einschränkt.

## Patentansprüche

1. Sterilisator (6) mit einer Sterilisierkammer (7) und mit einer Prüfeinrichtung (1) zur Prüfung der Effektivität eines Sterilisationsprozesses, die einen Prüfkörper (2) und eine Sonde (3) umfasst, wobei die Sonde (3, 3') im Betrieb mit dem Prüfkörper (2) verbunden ist, wobei der Prüfköper (2) außerhalb und die Sonde (3, 3') mindestens teilweise innerhalb der Sterilisierkammer (7) angeordnet ist und wobei die Prüfeinrichtung (1) mit der Sterilisierkammer (7) fest verbindbar ist **dadurch gekennzeichnet,**
**dass** eine Kühleinrichtung (4) zur Kühlung des Prüfkörpers (2) vorgesehen ist.

2. Sterilisator (6) nach Anspruch 1, **dadurch gekennzeichnet, dass** die Kühleinrichtung (4) als passive Kühleinrichtung ausgestaltet ist, welche vorzugsweise durch freie Konvektion erfolgt.

3. Sterilisator (6) nach Anspruch 1, **dadurch gekennzeichnet, dass** die Kühleinrichtung als aktive Kühleinrichtung (4) zur geregelten Kühlung des Prüfkörpers (2) ausgestaltet ist und eine Steuerungseinrichtung vorgesehen ist, die mit der aktiven Kühleinrichtung (4) zur Steuerung der Kühlleistung verbindbar ist.

4. Sterilisator (6) nach Anspruch 3, **dadurch gekennzeichnet, dass** die aktive Kühleinrichtung (4) ein Kühlmedium, beispielsweise Wasser- und/oder Luft, und/oder eine Kühlung durch Peltier-Elemente und/oder eine Einrichtung für die Erzeugung einer erzwungenen Konvektion umfasst.

5. Sterilisator (6) nach einem der Ansprüche 3 bis 4, **dadurch gekennzeichnet, dass** mittels der Steuerungseinrichtung die Temperatur des Prüfkörpers (2) einstellbar ist.

6. Sterilisator (6) nach einem der Ansprüche 1 bis 5, **dadurch gekennzeichnet, dass** die Sonde (3) als Hohlkörper zur Leitung von Dampf und/oder Agens und/oder Gasen ausgestaltet ist.

7. Sterilisator (6) nach einem der Ansprüche 1 bis 6, **dadurch gekennzeichnet, dass** am Prüfkörper (2) ein Temperatursensor (5) angeordnet ist.

8. Sterilisator (6) nach einem der Ansprüche 1 bis 7, **dadurch gekennzeichnet, dass** die Sonde (3) im oberen, unteren oder seitlichen Bereich der Sterilisierkammer (7) angeordnet ist.

9. Verfahren zum Betreiben eines Sterilisators (6) nach einem der Ansprüche 1 bis 8, **dadurch gekennzeichnet, dass** die Prüfeinrichtung (1) mittels einer Sterilisatorsteuerung automatisch mit der Wahl eines Sterilisierprogramms aktiviert wird, so dass ein Prüfprogramm gestartet wird.

10. Verfahren nach Anspruch 9, **dadurch gekennzeichnet, dass** durch die Prüfeinrichtung (1) während des Prüfprogramms ermittelten Daten an ein Chargendokumentationssystem übermittelt werden und/oder durch dieses ausgewertet und/oder archiviert werden.

11. Verfahren nach Anspruch 9 oder 10, **dadurch gekennzeichnet, dass** durch die Aktivierung der Prüfeinrichtung (1) mittels der Sterilisatorsteuerung ein vorgegebenes Programm, vorzugsweise eine Leerprüfung des Sterilisators (6), zeitverzögert durchgeführt wird.

12. Verfahren nach Anspruch 11, wobei eine Leerprüfung des Sterilisators (6) zeitverzögert durchgeführt wird, **dadurch gekennzeichnet, dass** mittels der Sterilisatorsteuerung eine Folge von Programmen einstellbar ist, die nach der Aktivierung automatisiert abläuft und dass die Leerprüfung Teil der Folge ist.

13. Vorrichtung zur Prüfung der Effektivität eines Sterilisationsprozesses, mit einer Prüfeinrichtung (1), wobei die Prüfeinrichtung (1) einen Prüfkörper (2) und eine Sonde (3, 3') umfasst, wobei die Sonde (3, 3') im Betrieb mit dem Prüfkörper (2) verbunden ist, **dadurch gekennzeichnet,**
**dass** eine Kühleinrichtung (4) zur Kühlung des Prüfkörpers (2) vorgesehen ist.

## Claims

1. A sterilizer (6) having a sterilization chamber (7) and having a testing device (1) for testing the effectiveness of a sterilization process, the testing device comprising a test object (2) and a probe (3), wherein the probe (3, 3') is connected to the test object (2) during operation, wherein the test object (2) is arranged outside the sterilization chamber (7) and the probe (3, 3') is at least partially arranged inside the sterilization chamber (7), and wherein the testing device (1) is fixedly connectable to the sterilization chamber (7), **characterized in that**
there is provided a cooling device (4) for cooling the test object (2).

2. The sterilizer (6) of claim 1, **characterized in that** the cooling device (4) is designed as a passive cooling device which preferably works by free convection.

3. The sterilizer (6) of claim 1, **characterized in that** the cooling device is designed as an active cooling device (4) for regulated cooling of the test object (2), and that there is provided a control device which is connectable to the active cooling device (4) for controlling the cooling power.

4. The sterilizer (6) of claim 3, **characterized in that** the active cooling device (4) comprises a cooling medium, such as water and/or air, and/or cooling by Peltier elements and/or a device for effecting forced convection.

5. The sterilizer (6) of either of claims 3 to 4, **characterized in that** the temperature of the test object (2) is adjustable by means of the control device.

6. The sterilizer (6) of any of claims 1 to 5, **characterized in that** the probe (3) is designed as a hollow body for carrying steam and/or agent and/or gases.

7. The sterilizer (6) of any of claims 1 to 6, **characterized in that** there is a temperature sensor (5) arranged on the test object (2).

8. The sterilizer (6) of any of claims 1 to 7, **characterized in that** the probe (3) is arranged in the upper, lower, or lateral region of the sterilization chamber (7).

9. A method of operating a sterilizer (6) of any of claims 1 to 8, **characterized in that** the testing device (1) is activated automatically by means of a sterilizer control by selecting a sterilizing program so that a test program is started.

10. The method of claim 9, **characterized in that** data obtained by the testing device (1) during the test program is transmitted to a batch documentation system and/or analyzed and/or archived by the latter.

11. The method of claim 9 or claim 10, **characterized in that** by activating the testing device (1) by means of the sterilizer control, a predefined program, preferably a test of the empty sterilizer (6), is executed with a time delay.

12. The method of claim 11, wherein a test of the empty sterilizer (6) is performed with a time delay, **characterized in that** by means of the sterilizer control it is possible to set a sequence of programs which runs automatically after activation, and that the test of the empty sterilizer is a part of the sequence.

13. An apparatus for testing the effectiveness of a sterilization process, the apparatus having a testing device (1), wherein the testing device (1) comprises a test object (2) and a probe (3,3'), wherein the probe (3, 3') is connected to the test object (2) during operation, **characterized in that**
there is provided a cooling device (4) for cooling the test object (2).

## Revendications

1. Stérilisateur (6) avec une chambre de stérilisation (7) et un dispositif de contrôle (1) en vue du contrôle de l'efficacité d'un processus de stérilisation, qui comprend un corps de contrôle (2) et une sonde (3), la sonde (3, 3') étant reliée en service avec le corps de contrôle (2), le corps de contrôle (2) étant disposé en dehors et la sonde (3, 3') au moins partiellement à l'intérieur de la chambre de stérilisation (7) et le dispositif de contrôle (1) étant reliable de manière fixe avec la chambre de stérilisation (7), **caractérisé**
**en ce qu'**un dispositif de refroidissement (4) est prévu en vue du refroidissement du corps de contrôle (2).

2. Stérilisateur (6) selon la revendication 1, **caractérisé en ce que** le dispositif de refroidissement (4) est conçu en dispositif de refroidissement passif, qui est réalisé de préférence par convection libre.

3. Stérilisateur (6) selon la revendication 1, **caractérisé en ce que** le dispositif de refroidissement est conçu en dispositif de refroidissement actif (4) en vue du refroidissement régulé du corps de contrôle (2), et **en ce qu'**un dispositif de commande est prévu, qui est reliable avec le dispositif de refroidissement actif (4) en vue de la commande de la puissance de refroidissement.

4. Stérilisateur (6) selon la revendication 3, **caractérisé en ce que** le dispositif de refroidissement actif (4) comprend un fluide de refroidissement, par exemple de l'eau et/ou de l'air, et/ou un refroidissement par élément Peltier et/ou un dispositif destiné à la production d'une convection forcée.

5. Stérilisateur (6) selon une quelconque des revendications 3 à 4, **caractérisé en ce que**, à l'aide du dispositif de commande, la température du corps de contrôle (2) est réglable.

6. Stérilisateur (6) selon une quelconque des revendications 1 à 5, **caractérisé en ce que** la sonde (3) est conçue en corps creux en vue de la conduite de vapeur et/ou d'agent chimique et/ou des gaz.

7. Stérilisateur (6) selon une quelconque des revendications 1 à 6, **caractérisé en ce que** sur le corps de contrôle (2), un capteur de température (5) est disposé.

8. Stérilisateur (6) selon une quelconque des revendications 1 à 7, **caractérisé en ce que** la sonde (3) est disposée dans la zone supérieure, inférieure ou latérale de la chambre de stérilisation (7).

9. Procédé en vue de l'exploitation d'un stérilisateur (6) selon une quelconque des revendications 1 à 8, **caractérisé en ce que** le dispositif de contrôle (1) est activé automatiquement au moyen d'une commande de stérilisateur avec la sélection d'un programme de stérilisation, de sorte qu'un programme de contrôle (1) est démarré.

10. Procédé selon la revendication 9, **caractérisé en ce que** les données relevées par le dispositif de contrôle (1) pendant le programme de contrôle sont transmises à un système de documentation de charges et/ou évaluées et/ou archivées par celui-ci.

11. Procédé selon les revendications 9 ou 10, **caractérisé en ce que**, par l'activation du dispositif de contrôle (1) au moyen de la commande de stérilisateur, un programme préconisé, de préférence un contrôle à vide du stérilisateur (6), est réalisé avec retard de temps.

12. Procédé selon la revendication 11, un contrôle à vide du stérilisateur (6) étant réalisé avec retard de temps, **caractérisé en ce que**, au moyen de la commande de stérilisateur, une séquence de programmes est réglable, qui se déroule de manière automatisée après l'activation et **en ce que** le contrôle à vide fait partie de la séquence.

13. Dispositif en vue du contrôle de l'efficacité d'un processus de stérilisation, avec un dispositif de contrôle (1), le dispositif de contrôle (1) comprenant un corps de contrôle (2) et une sonde (3, 3'), la sonde (3, 3') étant reliée en service avec le corps de contrôle (2), **caractérisé**
**en ce qu'**un dispositif de refroidissement (4) est prévu en vue du refroidissement du corps de contrôle (2).
